# EUROPEAN PATENT SPECIFICATION

(11) **EP 3 232 925 B1**
(45) Date of publication and mention of the grant of the patent: **04.09.2019**
(21) Application number: 15804473.5
(22) Date of filing: 03.12.2015
(51) Int. Cl.: A61B 5/11, A61B 5/00

(54) **ACTIVITY CLASSIFICATION AND COMMUNICATION SYSTEM FOR WEARABLE MEDICAL DEVICE**
AKTIVITÄTSKLASSIFIZIERUNG UND KOMMUNIKATIONSSYSTEM FÜR EINE TRAGBARE MEDIZINISCHE VORRICHTUNG
CLASSIFICATION D'ACTIVITÉ ET SYSTÈME DE COMMUNICATION DE DISPOSITIF MÉDICAL PORTABLE

(30) Priority: 18.12.2014 EP 14198731
(43) Date of publication of application: 25.10.2017
(73) Proprietor: Koninklijke Philips N.V., 5656 AE Eindhoven (NL)
(72) Inventor: HARMA, Aki Sakari, 5656 AE Eindhoven (NL)
(74) Representative: de Haan, Poul Erik
(86) International application number: PCT/EP2015/078435
(87) International publication number: WO 2016/096443

(56) References cited:
- EP-A1- 2 704 098
- WO-A1-2012/089278
- US-A1- 2011 218 462
- US-A1- 2013 060 480
- US-A1- 2013 278 076

## Description

### FIELD OF INVENTION

The invention relates to a method for transmitting activity information from a wearable medical device to a patient monitoring system, a wearable medical device and a patient monitoring system.

### BACKGROUND OF THE INVENTION

Wearable medical devices are becoming more and more popular to monitor the health status of a patient. These wearable medical devices may comprise a sensor which is used to recognize the activities of a patient. For example, US 8,374,775 B2 discloses a method for classifying activity states using sensor measurements. Typically, the current activity state that a patient performs is wirelessly transmitted to a central patient monitoring system, where it allows care-keeping personnel to keep track of the patient's activity, health status and treatment success. However, some regular activities of the patient (e.g. walking or toileting) may take place outside of the patient's room, where it may not be possible to establish a reliable wireless connection between the wearable medical device and the patient monitoring system. Accordingly, important information on the patient's activities may be lost.

US 2013/060480 A1 describes a method for adaptive display and filtering of sensors and sensor data. A sensor manager processes sensor signals for comparison against predetermined signals. The sensor manager determines one or more parameters for one or more filters based, at least in part, on the comparison, wherein the one or more filters operate, at least in part, on the one or more sensors, one or more other signals determined form the one or more sensors, or a combination thereof.

WO 2012/089278 A1 relates to a method of compressing data output from an acceleration measurement means configured to be transported, carried or worn by a user.

EP 2704098 A1 relates to a method of confirming motion parameters, an apparatus for the same, and a motion assisting device. Motion data of a recognized object comprises the acceleration of the recognized object sampled by a tri-axial accelerometer, the angular velocity of the recognized object sampled by a tri-axial gyroscope, and an angle of the recognized object sampled by a tri-axial magnetometer.

US 2011/218462 A1 relates to a system for measuring and analyzing movement or force in conjunction with sports, physical fitness or therapy.

US 2013/278076 A1 relates to a telemetry system with a wireless power receiver and monitoring devices that each have one or more sensors and a unique user ID. The one or more sensors acquire user information selected from of at least one of, a user's activities, behaviors and habit information.

### SUMMARY OF THE INVENTION

Hence, it may be an object of the present invention to provide a method for transmitting activity information from a wearable medical device to a patient monitoring system, a wearable medical device and a patient monitoring system allowing for a more reliable checking of the current status of a patient by care personnel.

Said object has been addressed with the method for transmitting activity information from a wearable medical device to a patient monitoring system, a wearable medical device and a patient monitoring system according to the independent claims. Advantageous embodiments of the method for transmitting activity information from a wearable medical device to a patient monitoring system are described in the dependent claims. Wearable medical devices and patient monitoring systems may be adapted accordingly.

According to an aspect, a method for transmitting activity information from a wearable medical device to a patient monitoring system is provided, wherein the method comprises generating an activity data packet, wherein the activity data packet comprises at least a first activity field indicative of a recent activity and a second activity field indicative of a past activity, and transmitting, in particular wirelessly transmitting, the activity data packet from the wearable medical device to the patient monitoring system.

Providing within one activity data packet not only an activity field indicative of a most recent activity but also a second activity field indicative of a past activity that occurred before the most recent activity may allow for a more continuous monitoring of a patient in case activity data packets get lost. Further, sending a second activity field indicative of such a past activity may allow for correcting the assumption of the past activity. In a conventional system, a possibly incorrect activity may be indicated to the patient monitoring system. Sending a second activity field indicative of a past activity may allow for correcting said erroneous assessment. For example, according to a first activity data packet sent to the patient monitoring system, the patient may have been sitting in a chair at time t. However, when assessing the next activity at time t+1, it may be found that at time t+1 the patient is lying in bed. Hence, the second data packet may have a corrected second activity field indicating that the patient at time t has not been sitting in the chair but on the bed.

Therefore, in the improved communication of patient activity information to the patient monitoring system it would be able to change activity history that has already been transmitted.

Arranging the first activity field within an activity data packet before the second activity field may allow for transmitting information indicative of a most recent activity before information indicative of a past activity that occurred earlier. If, for example, the first activity field indicates that the patient wearing the wearable medical device has fallen out of bed, upon reception of the first activity field immediate reaction may be required from the nurses. Hence, transmitting the first activity field before the second activity field may allow for important recent information to be considered even if not the whole data packet is transmitted.

Transmitting the activity data packet may comprise transmitting the activity data packet from every 10 seconds allowing for an immediate response from the personnel to every 2 minutes leading to reduced energy consumption for transmission. A good compromise may in particular be achieved if the activity data packet is transmitted once in a minute.

In a first embodiment of the method for transmitting activity information from a wearable medical device to a patient monitoring system, the activity data packet comprises a header field, wherein the header field comprises a first header subfield indicative of a time range represented by an activity field and/or a second header subfield indicative of the number of activity fields comprised within the activity data packet.

Providing a first header subfield indicative of a time range represented by an activity field may allow for a more flexible transmission of activity data. The first header subfield may in particular indicate that for a given activity data packet each activity field relates to a time period of 10 seconds. For another activity data packet, the value of the first header subfield may specify that each activity field relates to a time period of 120 seconds, i.e. 2 minutes.

A second header subfield indicative of the number of activity fields comprised within the activity data packet may make it possible to detect the end of an activity data packet without providing a special end-of-file character. Alternatively or in addition, the second header subfield indicative of the number of activity fields comprised within the activity data packet may also allow for verifying if all activity fields have been received.

In another embodiment of the method for transmitting activity information from a wearable medical device to a patient monitoring system, transmitting the activity data packet from the wearable medical device to the patient monitoring system comprises acknowledge-free transmitting of the activity data package.

In a hospital environment, multiple wearable medical devices may communicate with one or several patient monitoring systems leading to noisy wireless channels. Dispensing with the transmission of acknowledged signals by the patient monitoring system upon receipt of activity data packets from the wearable medical devices may help to reduce the congestion of the wireless channels.

Further, an embodiment of the method for transmitting activity information from a wearable medical device to a patient monitoring system prescribes that the first activity field and the second activity field each comprise a first activity subfield indicative of an activity type and a second activity subfield indicative of a certainty of the activity type. The certainty of the activity type may be considered as the likelihood an activity type has correctly been identified. The second activity subfield may get updated based on preceding or following activities.

A patient monitoring system may provide more reliably information about a patient's health and recovering status upon receipt of a first activity field and a second activity field each comprising a first activity subfield indicative of an activity type and a second activity subfield indicative of a certainty of the activity type.

Moreover, in an embodiment of the method for transmitting activity information from a wearable medical device to a patient monitoring system, generating the data packet comprises interleaving at least one interleaved activity field based on reasoning, wherein the interleaved activity field comprises a first activity subfield indicative of an activity type and second activity subfield indicative of a certainty of the activity type.

Some activity types relevant for a person's health and/or recovering status may be difficult to derive directly from sensor data. Interleaving an interleaved activity field based on reasoning, wherein the interleaved activity field comprises a first activity subfield indicative of an activity type and a second activity subfield indicative of a certainty of the activity type, may provide additional information on the patient valuable for treatment purposes.

In another embodiment of the method for transmitting activity information from a wearable medical device to a patient monitoring system, generating the activity data packet comprises interleaving at least one interleaved activity field based on reasoning, wherein the interleaved activity field comprises a first activity sub field indicative of an event and a second activity subfield indicative of a certainty of the event.

Some events happening to a user of a wearable medical device may require special handling by a patient monitoring system. Deriving said events based on reasoning, in particular based on the activity type derived from the first activity field and the second activity field, may allow for an improved medical care of a patient.

It may also be possible that the second activity subfield is indicative of a severity of the event. For example, the event may be a fall on the stairs, which requires immediate help from the care-keeping professionals in a hospital.

The embodiment may also comprise sending an immediate activity data packet in case of special events or in case of a high severity. The immediate activity data packet may be sent in addition to an activity data packet sent, for example, every minute. The immediate activity data packet may also trigger a shortening of the transmission period.

Further, an embodiment of the method for transmitting activity information from a wearable medical device to a patient monitoring system may prescribe that generating the activity data packet comprises interleaving at least one interleaved activity field based on reasoning, wherein the interleaved activity field comprises a first activity subfield indicative of an attribute of an activity type indicated in the first activity field and a second activity subfield indicative of a value of the attribute.

Improvements of a patient's health status may not only be related to the activity types the patient performs but also to certain events, e.g., a fall from walking or from bed. Events detected based on changes in an activity type (e.g. from lying in bed to lying on the floor) and included in the activity data packet may trigger special warnings when transmitted to a patient monitoring system.

In another embodiment of the method for transmitting activity information from a wearable medical device to a patient monitoring system, the wearable medical device comprises an accelerometer and deriving the value of the first activity subfield comprises computing several features, in particular in different temporal ranges, from the raw accelerometer data, which features may characterize the orientation and movement of the wearable medical device. The particular activity type may in particular be recognized using statistical machine learning algorithms based on classification or regression of the vectors of feature values.

Moreover, in an embodiment of the method for transmitting activity information from a wearable medical device to a patient monitoring system, the wearable medical device comprises an accelerometer, in particular a 3-axis-accelerometer, and deriving the value of the first activity subfield comprises detecting the orientation of the wearable medical device based on raw accelerometer sensor data.

The orientation of the wearable medical device may give a very reliable indication of the activity type of the user. If the wearable medical device is, for example, a breast belt, the orientation of the breast belt may allow for differentiating between lying and standing.

In another embodiment of the method for transmitting activity information from a wearable medical device to a patient monitoring system, the wearable medical device comprises an accelerometer, in particular a 3-axis-accelerometer, and deriving the value of the first activity subfield comprises detecting the acceleration magnitude of the wearable medical device within a short time frame based on the raw sensor data.

A high acceleration magnitude within a short time frame, in particular within 20 seconds, more particularly within 5 seconds, may be a further factor to establish the activity type. For example, a high acceleration magnitude may indicate shivering of a patient using the wearable medical device.

Moreover, in an embodiment of the method for transmitting activity information from a wearable medical device to a patient monitoring system, the wearable medical device comprises a sensor system, in particular an accelerometer, more particularly a 3-axis-accelerometer, and deriving the value of the first activity subfield comprises detecting a periodicity and/or a cadence in the raw sensor data.

Detecting a periodicity in the raw sensor data may be useful to determine, for example, the step regularity of a walking patient. The step regularity may be a good indicator how a patient recovers from a hip replacement.

In another embodiment of the method for transmitting activity information from a wearable medical device to a patient monitoring system, deriving an activity type and/or reasoning comprises using a naive Bayes model.

Classifying using a naive Bayes model may require only a small amount of training data to estimate the parameters necessary for classification.

Further, an embodiment of the method for transmitting activity information from a wearable medical device to a patient monitoring system prescribes deriving an activity type and/or reasoning comprises classifying based on a machine learning algorithm performing a quadratic discriminant analysis or a linear discriminant analysis.

A linear discriminant analysis may allow for determining activity types upon a linear combination of features derived from the raw sensor data.

Activity types being represented by a non-linear combination of features may be more easily distinguished with a quadratic discriminant analysis.

Moreover, in an embodiment of the method for transmitting activity information from a wearable medical device to a patient monitoring system, deriving an activity type and/or reasoning comprises classifying with a machine learning algorithm using a neural network.

Using a neural network may not require making assumptions about the correlation of features extracted from the raw sensor data.

It may be advantageous to use multiple classification algorithms when performing the method for transmitting activity information from a wearable medical device to a patient monitoring system so that some of them are used for detecting a particular stationary activity type and other are detectors of particular events (e.g., a fall detector).

The activity type and its certainty may depend on previous or following activity types and their likelihood values. Typically, in a classification algorithm, a likelihood of a class in a sequence of observations may be formulated by conditional probabilities or using Markov chains (or Markov random fields), which may describe transition probabilities from one state to another. The reasoning using, for example, a Markov chain model of a sequence of activity types may lead to a change in the activity type classification of the most recent or a previous activity type.

According to a further aspect, a wearable medical device is provided, wherein the wearable medical device comprises a sensor system, in particular an accelerometer, more particularly a 3-axis-accelerometer, a classifier for generating an activity data packet, wherein the activity data packet comprises at least a first activity field indicative of a recent activity and a second activity field indicative of a past activity, and a device communication unit for transmitting the activity data packet to a patient monitoring system. Another aspect relates to a patient monitoring system comprising a system communication unit receiving and processing an activity data packet comprising at least a first activity field indicative of a recent activity and a second activity field indicative of a past activity.

### BRIEF DESCRIPTION OF THE DRAWINGS

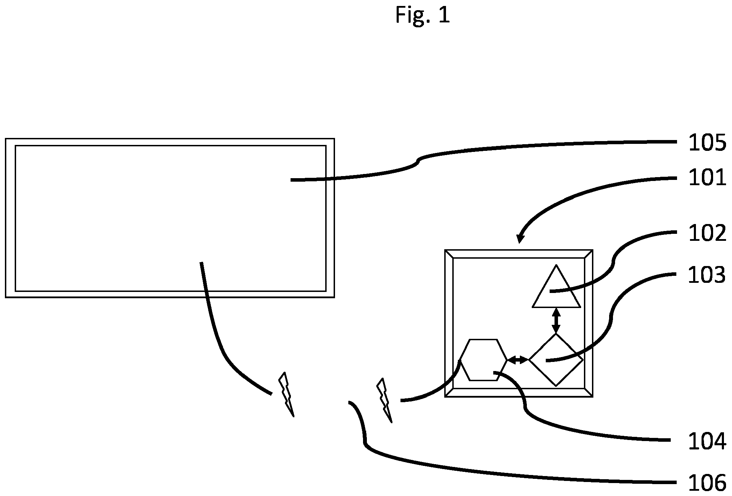

In the drawing
Fig.1 shows a wearable medical device and a patient monitoring system.

### DETAILED DESCRIPTION OF EMBODIMENTS

Figure 1 shows a wearable medical device 101 comprising a sensor system 102, more specifically a 3-axis accelerometer, a classifier 103 and a device communication unit 104. Activity data packets may be sent from the wearable medical device 101 to a patient monitoring system 105 over a wireless connection 106.

The wireless connection 106 may be based on packet-switched near-field radio. A typical data packet for a packet-switched near-field radio may have the structure according to table 1. Therein, PHY relates to the physical layer, MAC to media access control, i.e. the data link layer, NET to the network layer and MIC to a message integrity code according to the Open Systems Interconnection model (OSI).

**Table 1**

| PHY | MAC | NET | Application Payload | MIC |
|---|---|---|---|---|
| | | | max 90 bytes | |
| max 132 bytes | | | | |

According to the exemplary structure pursuant to table 1, the application payload, i.e. the effective payload, is at maximum 90 bytes. Pursuant to an embodiment of the method, a format of the application payload, i.e. an activity data packet, may have the format as shown in table 2.

**Table 2**

| T | N | AF(1) | ... | AF(n-1) | AF(n) | AF(n+1) | ... | AF(N) |
|---|---|---|---|---|---|---|---|---|
| max 86 bytes | | | | | | | | |

As shown, the activity data packet may comprise header information T, N and activity fields AF. The field T in the header refers to a time range the activity information corresponds to (e.g., 1 second, 1 minute, etc.). The field N may indicate the number of activity fields comprised within the activity data packet. The first activity field AF(1) may correspond to the most recent time period. The activity data packet may comprise additional activity fields AF(n) relating to past time periods.
Table 3 shows an exemplary activity data packet in bitstream syntax, wherein ActivityClassifierPacket relates to an activity data packet, acFieldType to the field T, acNumberofFields to the field N and acActivityType as well as acActivityStrength to an activity field as described hereinbefore.

**Table 3**

| Syntax | No. of bytes | Mnemonic |
|---|---|---|
| ActivityClassifierPacket() | | |
| { | | |
| acFieldType; | 1 | u_8 |
| acNumberOfFields; | 1 | u_8 |
| for (n = 0; n < acNumberOfFields; n++) | | |
| { | | |
| acActivityType[n]; | 1 | u_8 |
| acActivityStrength[n]; | 1 | u_8 |
| } | | |
| } | | |

The first field of the activity data packet, acFieldType, is an indicator, which describes the time range each activity field (acActivityType, acActivityStrength) represents. The time ranges may be coded as proposed in table 4 (the suffix h here and in the following indicates hexadecimal notation).

**Table 4**

| Hex | 00h | 01h | 02h | 03h | 04h | 05h | 06h | 07h | 08h | 09h | 0Ah | 0Bh | 0Ch | 0Dh | 0Eh | 0Fh |
|---|---|---|---|---|---|---|---|---|---|---|---|---|---|---|---|---|
| t [s] | 0 | 0.5 | 1 | 2 | 5 | 8 | 10 | 30 | 60 | 90 | 120 | 300 | 600 | 900 | 1800 | 3600 |

As shown, the first 16 possible values represent activity field durations in seconds. The selection 00h may represent a special case, in which the activity fields all correspond to simultaneous current activities. The further 240 possible values are reserved for later use and allow for an adaption of the activity data packet for future developments. Accordingly, the respective remaining four bits are masked.

The number N of activity fields may be limited to 42. The activity fields may, as shown in table 3, be split into a first sub field acActivityType and a second sub field acActivityStrength. Each sub field may be a one byte sub field. It is possible to represent 256 activity types by one byte, which may be split into groups including activities, events and attributes, for example according to table 5.

**Table 5**

| Code | Activity / Event / Attribute |
|---|---|
| 00h | Device on table |
| 01h | Device loose |
| 02h | Unknown / Uncertain |
| | |
| 11h | Lying |
| 12h | Rolling |
| 13h | Restless lying |
| | |
| 20h | Seizure / jerks |
| 21h | Shivering |
| 22h | Sleeping |
| | |
| 30h | Sit |
| 31h | Eat |
| 32h | Drink |
| 33h | Communicating / Talking |
| 34h | Reading / Using a tablet computer |
| 35h | Physical exercise |
| | |
| 50h | Standing |
| 51h | Walking |
| 52h | Walking with crutches |
| 53h | Walking with assistance |
| 54h | Walking with support (walker) |
| 55h | Walking using rail |
| 56h | Walking in treadmill |
| | |
| 60h | Wheelchair (self-propelled) |
| 61h | Wheelchair (pushed by someone) |
| | |
| 90h | Walking stairs down |
| 91h | Walking stairs up |
| 92h | Elevator |
| | |
| A0h | Toileting |
| A1h | Showering |
| A2h | Gymnastics / Rehabilitation |
| | |
| B0h | Fall from walking |
| B1h | Fall from bed |
| | |
| C0h | Bed exit |
| C1h | Early bed exit |
| C2h | Bed entry |
| | |
| CAh | Collision |
| | |
| D0h | Step rate |
| D1h | Step regularity |
| D2h | Stability / balance |
| D3h | Symmetry in ambulating |
| D4h | Impact in steps |
| | |
| E0h | Awakeness |
| | |
| FFh | Place |

In table 5, activity types coded from 00h to AFh may represent activities, those from B0h to CFh events and those from D0h to FFh attributes. The second subfield of an activity field relating to an activity may indicate the certainty that the activity has been correctly identified. Attributes may refer to the preceding activity. For example, if an activity field indicates the activity type "walking", the following activity field may provide attribute for this "walking" activity such as the step rate. Accordingly, the activity field may indicate in the first subfield the type of the attribute, e.g. the step rate, and in the second subfield a value for said attribute, e.g. the step rate in steps per minute. A special attribute coded FFh may indicate the place or location where the preceding activity has taken place. In this case, the second subfield may indicate the place according to the location codes shown in table 6.

**Table 6**

| Hex | 00h | 01h | 02h | 03h | 04h | 05h | 06h | 07h |
|---|---|---|---|---|---|---|---|---|
| Place | Bed | Patient room | Hallway | Waiting room | Treatment room | Cafe | Toilet | Shower |

In an example according to table 7, an activity data packet may comprise six activity fields (N = 06h) each representing a time period of 10 seconds (T = 06h), of which only the first three activity fields are shown.

**Table 7**

| T | N | AF(1,T) | AF(1,S) | AF(2,T) | AF(2,S) | AF(3,T) | AF(3,S) | ... |
|---|---|---|---|---|---|---|---|---|
| 06h | 06h | 50h | 52h | 30h | D4h | 11h | f0h | ... |

The first activity field AF(1) indicates that the most recent activity has been standing (AF(1,T) = 50h) with a certainty of 52h, which has been preceded by sitting (AF(2,T) = 30h), which has been preceded by lying (AF(3,T) = 11h).

Based on reasoning, it may be determined that in going from lying to walking the patient must have performed a bed exit and that the sitting took place in bed because it occurred right before a bed exit.

Hence, as shown in table 8, the activity data packet for the next time period may be augmented with this additional information.

**Table 8**

| T | N | AF(1,T) | AF(1,S) | AF(2,T) | AF(2,S) | AF(3,T) | AF(3,S) | AF(4,T) | AF(4,S) | AF(5,T) | AF(5,S) | ... |
|---|---|---|---|---|---|---|---|---|---|---|---|---|
| 06h | 06h | 51h | 80h | 50h | 52h | C0h | DDh | 30h | d4h | FFh | 00h | ... |

As shown, the most recent activity changed from standing (AF(2,T) = 50h) to walking (AF(1,T) = 51h). Moreover, interleaved activity fields AF(3) and AF(5) have been interleaved indicating the bed exit event (AF(3,T) = C0h) and that sitting took place in bed (AF(5,T) = FFh, AF(F,S) = 00h).

Said additional information may be valuable for a better treatment of the patient.

In an embodiment, a number of numerical features may be computed from the raw accelerometer data provided by the sensor system 102. These features may, for example, relate to the orientation of the wearable medical device, an acceleration magnitude within a short time frame, a periodicity or cadence in the raw data or other physical time-series. A classifier based on a machine learning algorithm may be used to derive an activity type from the numerical features. More particularly, the classifier may be based on a naive Bayes classifier principle and be trained using a collection of manually annotated activity data from different activity types from several patients (e.g., lying in bed probably asleep, moving actively in bed, sitting, standing, walking, ambulating using a wheelchair).

A classifier using the naive Bayes classifier principle may provide a likelihood score for each of the activity types. The winning activity type may be selected as activity type corresponding to the current time frame. Typically, the duration of a time-frame of an activity type analysis may be one second.

## Claims

1. Method for transmitting activity information from a wearable medical device (101) to a patient monitoring system (105), wherein the method comprises:
generating an activity data packet, wherein the activity data packet comprises a least
- a first activity field indicative of a recent activity and
- a second activity field indicative of a past activity; and
transmitting the activity data packet from the wearable medical device (101) to the patient monitoring system (105); and
wherein the first activity field and the second activity field each comprise:
- a first activity subfield indicative of an activity type and
- a second activity subfield indicative of a certainty of the activity type.

2. Method according to claim 1, wherein the activity data packet comprises a header field,
wherein the header field comprises
- a first header subfield indicative of a time range represented by an activity field; and/or
- a second header subfield indicative of the number of activity fields comprised within the activity data packet.

3. Method according to claim 1 or 2,
wherein transmitting the activity data packet from the wearable medical device to the patient monitoring system comprises acknowledge-free transmitting of the activity data package.

4. Method according to any one of claims 1 to 3,
wherein generating the data packet comprises interleaving at least one interleaved activity field based on reasoning and
wherein the interleaved activity field comprises a first activity subfield indicative of an activity type and a second activity subfield indicative of a certainty of the activity type.

5. Method according to any one of claims 1 to 4,
wherein generating the activity data packet comprises interleaving at least one interleaved activity field based on reasoning and
wherein the interleaved activity field comprises a first activity subfield indicative of an event and a second activity subfield indicative of a certainty of the event.

6. Method according to any one of claims 1 to 5,
wherein generating the activity data packet comprises interleaving at least one interleaved activity field based on reasoning and
wherein the interleaved activity field comprises a first activity subfield indicative of an attribute of an activity type indicated in the first activity field and a second activity subfield indicative of a value of the attribute.

7. Method according to any one of claims 1 to 6,
wherein the wearable medical device comprises an accelerometer (102); and
wherein deriving the value of the first activity subfield comprises detecting the orientation of the wearable medical device based on raw accelerometer sensor data.

8. Method according to any one of claims 1 to 7,
wherein the wearable medical device comprises an accelerometer (102) and
wherein deriving the value of the first activity subfield comprises detecting the acceleration magnitude of the wearable medical device within a short time frame based on the raw sensor data.

9. Method according to any one of claims 1 to 8,
wherein the wearable medical device comprises a sensor system (102) and
wherein deriving the value of the first activity subfield comprises detecting a periodicity and/or a cadence in the raw sensor data.

10. Method according to any one of claims 1 to 9,
wherein deriving an activity type and/or reasoning comprises classifying using a naive Bayes model.

11. Method according to any one of claims 1 to 10,
wherein deriving an activity type and/or reasoning comprises classifying based on a machine learning algorithm performing a quadratic discriminant analysis or a linear discriminant analysis.

12. Method according to any one of claims 1 to 11,
wherein deriving an activity type and/or reasoning comprises classifying with a machine learning algorithm using a neural network.

13. Wearable medical device comprising
a sensor system (102);
a classifier (103) for generating an activity data packet, wherein the activity data packet comprises at least
- a first activity field indicative of a recent activity and
- a second activity field indicative of a past activity; and;
a device communication unit (104) for transmitting the activity data packet to a patient monitoring system (105); and
wherein the first activity field and the second activity field each comprise:
- a first activity subfield indicative of an activity type and
- a second activity subfield indicative of a certainty of the activity type.

14. Patient monitoring system (105) comprising
a system communication unit for receiving and processing of an activity data packet transmitted by a wearable medical device, the activity data packet comprising at least
- a first activity field indicative of a recent activity and
- a second activity field indicative of a past activity; and
wherein the first activity field and the second activity field each comprise:
- a first activity subfield indicative of an activity type and
- a second activity subfield indicative of a certainty of the activity type.

## Patentansprüche

1. Verfahren zum Übertragen von Aktivitätsinformationen von einer tragbaren medizinischen Vorrichtung (101) auf ein Patientenüberwachungssystem (105), wobei das Verfahren Folgendes umfasst:
Erzeugen eines Aktivitätsdatenpakets, wobei das Aktivitätsdatenpaket mindestens Folgendes umfasst
- ein erstes Aktivitätsfeld, das für eine kürzliche Aktivität indikativ ist, und
- ein zweites Aktivitätsfeld, das für eine vergangene Aktivität indikativ ist; und
Übertragen des Aktivitätsdatenpakets von der tragbaren medizinischen Vorrichtung (101) auf das Patientenüberwachungssystem (105); und
wobei das erste Aktivitätsfeld und das zweite Aktivitätsfeld jeweils Folgendes umfassen:
- ein erstes Aktivitätssubfeld, das für einen Aktivitätstyp indikativ ist, und
- ein zweites Aktivitätssubfeld, das für eine Bestimmtheit des Aktivitätstyps indikativ ist.

2. Verfahren nach Anspruch 1, wobei das Aktivitätsdatenpaket ein Header-Feld umfasst,
wobei das Header-Feld Folgendes umfasst
- ein erstes Header-Subfeld, das für einen durch ein Aktivitätsfeld repräsentierten Zeitraum indikativ ist; und/oder
- ein zweites Header-Subfeld, das für die Anzahl innerhalb des Aktivitätsdatenpakets enthaltener Aktivitätsfelder indikativ ist.

3. Verfahren nach Anspruch 1 oder 2,
wobei das Übertragen des Aktivitätsdatenpakets von der tragbaren medizinischen Vorrichtung auf das Patientenüberwachungssystem bestätigungsfreies Übertragen des Aktivitätsdatenpakets umfasst.

4. Verfahren nach einem der Ansprüche 1 bis 3,
wobei das Erzeugen des Datenpakets das Verschränken mindestens eines auf Logik basierenden verschränkten Aktivitätsfelds umfasst und
wobei das verschränkte Aktivitätsfeld ein erstes Aktivitätssubfeld, das für einen Aktivitätstyp indikativ ist, und ein zweites Aktivitätssubfeld, das für eine Bestimmtheit des Aktivitätstyps indikativ ist, umfasst.

5. Verfahren nach einem der Ansprüche 1 bis 4,
wobei das Erzeugen des Aktivitätsdatenpakets das Verschränken mindestens eines auf Logik basierenden verschränkten Aktivitätsfelds umfasst und
wobei das verschränkte Aktivitätsfeld ein erstes Aktivitätssubfeld, das für ein Ereignis indikativ ist, und ein zweites Aktivitätssubfeld, das für eine Bestimmtheit des Ereignisses indikativ ist, umfasst.

6. Verfahren nach einem der Ansprüche 1 bis 5,
wobei das Erzeugen des Aktivitätsdatenpakets das Verschränken mindestens eines auf Logik basierenden verschränkten Aktivitätsfelds umfasst und
wobei das verschränkte Aktivitätsfeld ein erstes Aktivitätssubfeld, das für ein Attribut eines in dem ersten Aktivitätsfeld angegebenen Aktivitätstyps indikativ ist, und ein zweites Aktivitätssubfeld, das für einen Wert des Attributs indikativ ist, umfasst.

7. Verfahren nach einem der Ansprüche 1 bis 6,
wobei die tragbare medizinische Vorrichtung einen Beschleunigungsmesser (102) umfasst; und
wobei das Ableiten des Werts des ersten Aktivitätssubfelds das Detektieren der Orientierung der tragbaren medizinischen Vorrichtung basierend auf Rohdaten des Beschleunigungsmessersensors umfasst.

8. Verfahren nach einem der Ansprüche 1 bis 7,
wobei die tragbare medizinische Vorrichtung einen Beschleunigungsmesser (102) umfasst und
wobei das Ableiten des Werts des ersten Aktivitätssubfelds das Detektieren der Beschleunigungsgrößenordnung der tragbaren medizinischen Vorrichtung innerhalb eines auf den Sensorrohdaten basierenden kurzen Zeitrahmens umfasst.

9. Verfahren nach einem der Ansprüche 1 bis 8,
wobei die tragbare medizinische Vorrichtung ein Sensorsystem (102) umfasst und
wobei das Ableiten des Werts des ersten Aktivitätssubfelds das Detektieren einer Periodizität und/oder einer Kadenz in den Sensorrohdaten umfasst.

10. Verfahren nach einem der Ansprüche 1 bis 9,
wobei das Ableiten eines Aktivitätstyps und/oder einer Logik das Klassifizieren unter Verwendung eines naiven Bayes-Modells umfasst.

11. Verfahren nach einem der Ansprüche 1 bis 10,
wobei das Ableiten eines Aktivitätstyps und/oder einer Logik das Klassifizieren basierend auf einem Algorithmus für Maschinenlernen, der eine quadratische Diskriminanzanalyse oder eine lineare Diskriminanzanalyse durchführt, umfasst.

12. Verfahren nach einem der Ansprüche 1 bis 11,
wobei das Ableiten eines Aktivitätstyps und/oder einer Logik das Klassifizieren mit einem Algorithmus für Maschinenlernen unter Verwendung eines neuronalen Netzwerks umfasst.

13. Tragbare medizinische Vorrichtung, umfassend
ein Sensorsystem (102);
einen Klassifizierer (103) zum Erzeugen eines Aktivitätsdatenpakets, wobei das Aktivitätsdatenpaket mindestens Folgendes umfasst
- ein erstes Aktivitätsfeld, das für eine kürzliche Aktivität indikativ ist, und
- ein zweites Aktivitätsfeld, das für eine vergangene Aktivität indikativ ist; und
eine Vorrichtungskommunikationseinheit (104) zum Übertragen des Aktivitätsdatenpakets auf ein Patientenüberwachungssystem (105); und
wobei das erste Aktivitätsfeld und das zweite Aktivitätsfeld jeweils Folgendes umfassen:
- ein erstes Aktivitätssubfeld, das für einen Aktivitätstyp indikativ ist, und
- ein zweites Aktivitätssubfeld, das für eine Bestimmtheit des Aktivitätstyps indikativ ist.

14. Patientenüberwachungssystem (105), umfassend
eine Systemkommunikationseinheit zum Empfangen und Verarbeiten eines durch eine tragbare medizinische Vorrichtung übertragenen Aktivitätsdatenpakets, wobei das Aktivitätsdatenpaket mindestens Folgendes umfasst
- ein erstes Aktivitätsfeld, das für eine kürzliche Aktivität indikativ ist, und
- ein zweites Aktivitätsfeld, das für eine vergangene Aktivität indikativ ist; und
wobei das erste Aktivitätsfeld und das zweite Aktivitätsfeld jeweils Folgendes umfassen:
- ein erstes Aktivitätssubfeld, das für einen Aktivitätstyp indikativ ist, und
- ein zweites Aktivitätssubfeld, das für eine Bestimmtheit des Aktivitätstyps indikativ ist.

## Revendications

1. Procédé de transmission d'informations d'activité d'un dispositif médical portable (101) à un système de surveillance patient (105), dans lequel le procédé comprend :
la génération d'un paquet de données d'activité, dans lequel le paquet de données d'activité comprend au moins
- un premier champ d'activité indiquant une activité récente et
- un second champ d'activité indiquant une activité passée ; et
la transmission du paquet de données d'activité du dispositif médical portable (101) au système de surveillance de patient (105) ; et
dans lequel le premier champ d'activité et le second champ d'activité comprennent chacun :
- un premier sous-champ d'activité indiquant un type d'activité et
- un second sous-champ d'activité indiquant une certitude de type d'activité.

2. Procédé selon la revendication 1, dans lequel le paquet de données d'activité comprend un champ d'en-tête,
dans lequel le champ d'en-tête comprend
- un premier sous-champ d'en-tête indiquant une plage temporelle représentée par un champ d'activité ; et/ou
- un second sous-champ d'en-tête indiquant le nombre de champs d'activité compris à l'intérieur du paquet de données d'activité.

3. Procédé selon la revendication 1 ou 2,
dans lequel la transmission du paquet de données d'activité du dispositif médical portable au système de surveillance patient comprend la transmission sans accusé de réception du paquet de données d'activité.

4. Procédé selon l'une quelconque des revendications 1 à 3,
dans lequel la génération du paquet de données comprend l'imbrication d'au moins un champ d'activité imbriqué sur la base du raisonnement et
dans lequel le champ d'activité imbriqué comprend un premier sous-champ d'activité indiquant un type d'activité et un second sous-champ d'activité indiquant une certitude du type d'activité.

5. Procédé selon l'une quelconque des revendications 1 à 4,
dans lequel la génération du paquet de données d'activité comprend l'imbrication d'au moins un champ d'activité imbriqué sur la base d'un raisonnement et
dans lequel le champ d'activité imbriqué comprend un premier sous-champ d'activité indiquant un événement et un second sous-champ d'activité indiquant une certitude de l'événement.

6. Procédé selon l'une quelconque des revendications 1 à 5,
dans lequel la génération du paquet de données d'activité comprend l'imbrication d'au moins un champ d'activité sur la base d'un raisonnement et
dans lequel le champ d'activité imbriqué comprend un premier sous-champ d'activité indiquant un attribut d'un type d'activité indiqué dans le premier champ d'activité et un second sous-champ d'activité indiquant une valeur de l'attribut.

7. Procédé selon l'une quelconque des revendications 1 à 6,
dans lequel le dispositif médical portable comprend un accéléromètre (102) ; et
dans lequel l'obtention de la valeur du premier sous-champ d'activité comprend la détection de l'orientation du dispositif médical portable sur la base de données de capteur d'accéléromètre brutes.

8. Procédé selon l'une quelconque des revendications 1 à 7,
dans lequel le dispositif médical portable comprend un accéléromètre (102) et
dans lequel l'obtention de la valeur du premier sous-champ d'activité comprend la détection de la magnitude d'accélération du dispositif médical portable dans un court cadre temporel sur la base des données de capteur brutes.

9. Procédé selon l'une quelconque des revendications 1 à 8,
dans lequel le dispositif médical portable comprend un système de capteur (102) et
dans lequel l'obtention de la valeur du premier sous-champ d'activité comprend la détection d'une périodicité et/ou d'une cadence dans les données de capteur brutes.

10. Procédé selon l'une quelconque des revendications 1 à 9,
dans lequel la dérivation d'un type d'activité et/ou d'un raisonnement comprend la classification en utilisant un modèle de Bayes naïf.

11. Procédé selon l'une quelconque des revendications 1 à 10,
dans lequel un type d'activité et/ou un raisonnement comprend la classification sur la base d'un algorithme d'apprentissage machine réalisant une analyse discriminante quadratique ou une analyse discriminante linéaire.

12. Procédé selon l'une quelconque des revendications 1 à 11,
dans lequel un type d'activité et/ou un raisonnement comprend la classification avec un algorithme d'apprentissage machine en utilisant un réseau neuronal.

13. Dispositif médical portable comprenant
un système de capteur (102) ;
un classificateur (103) pour générer un paquet de données d'activité, dans lequel le paquet de données d'activité comprend au moins
- un premier champ d'activité indiquant une activité récente, et
- un second champ d'activité indiquant une activité passée ; et ;
une unité de communication de dispositif (104) pour transmettre le paquet de données d'activité à un système de surveillance patient (105) ; et
dans lequel le premier champ d'activité et le second champ d'activité comprennent chacun :
- un premier sous-champ d'activité indiquant un type d'activité et
- un second sous-champ d'activité indiquant une certitude du type d'activité.

14. Système de surveillance patient (105) comprenant
une unité de communication système pour recevoir et traiter un paquet de données d'activité transmis par un dispositif médical portable, le paquet de données d'activité comprenant au moins
- un premier champ d'activité indiquant une activité récente et
- un second champ d'activité indiquant une activité passée ; et
dans lequel le premier champ d'activité et le second champ d'activité comprennent chacun :
- un premier sous-champ d'activité indiquant un type d'activité et
- un second sous-champ d'activité indiquant une certitude du type d'activité.
